# EUROPEAN PATENT APPLICATION

(11) **EP 3 192 774 A1**
(43) Date of publication of application: **19.07.2017**
(21) Application number: 15827309.4
(22) Date of filing: 01.08.2015
(51) Int. Cl.: C01G 45/12, C02F 1/50, C02F 1/72, C02F 1/78

(54) **WATER INCLUDING PERMANGANATE IONS, AND METHOD FOR PRODUCING SAME**

(30) Priority: 01.08.2014 JP 2014158262
(71) Applicant: National Institute of Advanced Industrial Science and Technology, Tokyo 100-8921 (JP)
(72) Inventor: TAKAHASHI Masayoshi, Tsukuba-shi Ibaraki 305-8569 (JP)
(74) Representative: Walcher, Armin
(86) International application number: PCT/JP2015/071895
(87) International publication number: WO 2016/017820

(57) **Abstract**

An object of the present invention is to provide a water containing permanganate ions that exist stably over a long period of time and a method for producing the water. A water containing permanganate ions of the present invention as a means for resolution is produced by dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³. The half-life of permanganate ions of the water containing permanganate ions of the present invention is 3 months or longer, for example, when the water filled in an airtight container under atmospheric pressure is stored under a temperature condition of 40°C, and has significantly different properties from a water containing permanganate ions that is produced by dissolving a permanganate compound in water, although the two waters share a common point of containing permanganate ions.

## Description

### Technical Field

The present invention relates to a water containing permanganate ions and a method for producing the water.

### Background Art

Permanganate ions (MnO₄⁻: VII) are well known to be used for various oxidation reactions as an oxidant, and a technique for purifying soil and groundwater utilizing the oxidative effect thereof is proposed (Patent Document 1). In addition, applications of permanganate ions for antimicrobial agents and deodorants are also known. However, when permanganate ions are prepared by dissolving a permanganate compound such as potassium permanganate in water, there is a problem in that permanganate ions are reduced and disappear in a short period of time.

### Prior Art Documents

### Patent Document

Patent Document 1: JP-A-2003-104727

### Summary of the Invention

Problems that the Invention is to Solve

Thus, an object of the present invention is to provide a water containing permanganate ions that exist stably over a long period of time and a method for producing the water.

### Means for Solving the Problems

As a result of intensive studies in view of the above problem, the present inventor has found that when prescribed amounts of a divalent manganese compound, an organic iron compound, and an inorganic salt are dissolved in a water with ozone-microbubbles generated using an ozone gas at a prescribed concentration, it is possible to allow permanganate ions to exist in the water stably over a long period of time.

A water containing permanganate ions of the present invention made on the basis of the above findings is, as described in claim 1, produced by dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³.

A water containing permanganate ions described in claim 2 is the water containing permanganate ions according to claim 1, in which the divalent manganese compound is at least one selected from the group consisting of manganese nitrate, manganese sulfate, and manganese chloride.

A water containing permanganate ions described in claim 3 is the water containing permanganate ions according to claim 1, in which the organic iron compound is at least one selected from the group consisting of iron ammonium citrate, iron fulvate, iron acetate, heme iron, iron dextran, diethylenetriaminepentaacetic acid iron sodium salt, diethylenetriaminepentaacetic acid iron ammonium salt, ethylenediaminetetraacetic acid iron sodium salt, ethylenediaminetetraacetic acid iron ammonium salt, iron triethylenetetramine, dicarboxymethylglutamic acid iron sodium salt, ferrous citrate, iron sodium citrate, iron oxalate, ferrous succinate, iron sodium succinate citrate, ferrous pyrophosphate, ferric pyrophosphate, iron lactate, ferrous gluconate, ferrous formate, ferric formate, potassium ferric ammonium oxalate, ferrous ascorbate, and sodium ferric edetate.

A water containing permanganate ions described in claim 4 is the water containing permanganate ions according to claim 1, in which the inorganic salt is at least one selected from the group consisting of sodium chloride, potassium chloride, magnesium chloride, and magnesium sulfate.

A water containing permanganate ions described in claim 5 is the water containing permanganate ions according to claim 1, in which the generation of ozone-microbubbles is achieved by using a microbubble generator that can generate microbubbles having a particle size of 5 to 50 µm.

A water containing permanganate ions described in claim 6 is the water containing permanganate ions according to claim 5, in which the microbubble generator is a microbubble generator that employs a two-phase flow swirling method or a pressurized dissolution method.

A water containing permanganate ions described in claim 7 is the water containing permanganate ions according to claim 1, in which the dissolution of the divalent manganese compound, the organic iron compound, and the inorganic salt in the water with ozone-microbubbles generated is performed at an elapsed time of 10 minutes or longer after the redox potential of the water with ozone-microbubbles generated, which increases, reaches at least +600 mV.

A water containing permanganate ions described in claim 8 is the water containing permanganate ions according to claim 1, in which when the water filled in an airtight container under atmospheric pressure is stored under a temperature condition of 40°C, the half-life of permanganate ions is 3 months or longer.

A water containing permanganate ions described in claim 9 is the water containing permanganate ions according to claim 1, in which when the water containing permanganate ions filled in an airtight container under atmospheric pressure is cryopreserved for 6 months or longer under a temperature condition of -20°C and then naturally thawed at a normal temperature (25°C), the water recovers the state of the water containing permanganate ions before the cryopreservation.

A water containing permanganate ions described in claim 10 is the water containing permanganate ions according to claim 1, which is not irritating to mammal skin, has no oral acute toxicity to mammals, and has a sterilizing effect and a deodorant effect.

A method for producing a water containing permanganate ions of the present invention comprises, as described in claim 11, dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³.

A method for producing a water containing permanganate ions described in claim 12 is the method for producing a water containing permanganate ions according to claim 11, in which the generation of ozone-microbubbles is achieved by using a microbubble generator that can generate microbubbles having a particle size of 5 to 50 µm.

A method for producing a water containing permanganate ions described in claim 13 is the method for producing a water containing permanganate ions according to claim 11, in which the dissolution of the divalent manganese compound, the organic iron compound, and the inorganic salt in the water with ozone-microbubbles generated is performed at an elapsed time of 10 minutes or longer after the redox potential of the water with ozone-microbubbles generated, which increases, reaches at least +600 mV.

### Effect of the Invention

According to the present invention, a water containing permanganate ions that exist stably over a long period of time and a method for producing the water can be provided.

### Mode for Carrying Out the Invention

A water containing permanganate ions of the present invention is produced by dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³.

In the production of the water containing permanganate ions of the present invention, at the beginning, a water with ozone-microbubbles generated is prepared. The generation of ozone-microbubbles in water may be achieved according to any technique known per se and may be performed using a microbubble generator that employs a two-phase flow swirling method or a pressurized dissolution method that can generate microbubbles having a particle size of 5 to 50 µm. In the case where a two-phase flow swirling method is employed, a vortex flow having a radius of 10 cm or less is forcibly caused using a rotator or the like, and a gas-liquid mixture containing ozone that is to be contained in microbubbles is struck against an obstacle, such as a wall surface, or against a fluid having a different relative velocity, whereby a gas body obtained in the vortex flow is dispersed under the process of distraction of the vortex. As a result, ozone-microbubbles desired can be generated. In addition, in the case where a pressurized dissolution method is employed, ozone that is to be contained in microbubbles is dissolved in water at a high pressure of 2 atm or more and then depressurizing to the atmospheric pressure. As a result, ozone-microbubbles can be generated from dissolved gas under supersaturated conditions. In this case, at the pressure reduction region, a large number of vortexes having a radius of 1 mm or less are generated utilizing the water flow and an obstacle, and a large number of gas-phase nuclei (bubble nuclei) are formed due to the oscillation of water molecules in the central region of the vortex flow. At the same time, following the supersaturated conditions, the gas body in water is diffused toward these bubble nuclei resulting in the growth of the bubble nuclei. As a result, ozone-microbubbles desired can be generated in a large amount. Incidentally, ozone-microbubbles generated by these methods are microbubbles having a particle size of 50 µm or less. The particle size has a peak at 10 to 15 µm as measured with a laser-light-blocking liquid particle counter (e.g., LiQuilaz-E20 manufactured by SPM Co., etc.), and the number of microbubbles in the peak region is 1000/mL or more (see JP-A-2000-51107, JP-A-2003-265938, etc., if necessary). The ozone gas used for generating ozone-microbubbles in water is a gas prepared at a concentration of 1 to 300 g/Nm³ using, for example, a commercially available oxygen source ozone generator. When an ozone gas having a concentration of less than 1 g/Nm³ is used, it is not possible to efficiently generate a large amount of ozone-microbubbles in water. On the other hand, it is difficult to prepare an ozone gas having a concentration exceeding 300 g/Nm³. Incidentally, the ozone gas may contain oxygen, nitrogen, or the like in addition to ozone.

Next, prescribed amounts of a divalent manganese compound, an organic iron compound, and an inorganic salt are dissolved in the water with ozone-microbubbles generated. By dissolving a divalent manganese compound in the water with ozone supplied in the form of microbubbles, divalent manganese ions can be converted to septivalent permanganate ions. It is important for the ozone previously supplied into the water for dissolving the divalent manganese compound to be in the form of microbubbles. For example, when ozone is supplied by bubbling with a common aeration tube, divalent manganese ions are not converted to permanganate ions, and trivalent manganese oxide (Mn₂O₃) or tetravalent manganese oxide (manganese dioxide: MnO₂) are produced and precipitated. Furthermore, it is also important to dissolve the divalent manganese compound after ozone is supplied into water in the form of microbubbles. When ozone is supplied in the form of microbubbles into a water in which a divalent manganese compound has been dissolved, divalent manganese ions are not converted to permanganate ions, and trivalent manganese oxide (Mn₂O₃) or tetravalent manganese oxide (manganese dioxide: MnO₂) are produced and precipitated. As the divalent manganese compound, a water soluble compound, such as manganese nitrate, manganese sulfate, and manganese chloride can be used (the compound may be a hydrate). The amount of the divalent manganese compound dissolved is 0.1 µM to 1 mM. When the amount dissolved is less than 0.1 µM, a sufficient amount of permanganate ions may not be produced. Meanwhile, when the amount exceeds 1 mM, trivalent manganese oxide (Mn₂O₃) or tetravalent manganese oxide (manganese dioxide: MnO₂) may be produced and precipitated. The amount of the divalent manganese compound dissolved is desirably 1 to 100 µM.

By dissolving an organic iron compound in the water with ozone-microbubbles generated, permanganate ions can exist in the water stably over a long period of time. Specific examples of the organic iron compound include water soluble compounds, such as iron ammonium citrate, iron fulvate, iron acetate, heme iron, iron dextran, diethylenetriaminepentaacetic acid iron sodium salt, diethylenetriaminepentaacetic acid iron ammonium salt, ethylenediaminetetraacetic acid iron sodium salt, ethylenediaminetetraacetic acid iron ammonium salt, iron triethylenetetramine, dicarboxymethylglutamic acid iron sodium salt, ferrous citrate, iron sodium citrate, iron oxalate, ferrous succinate, iron sodium succinate citrate, ferrous pyrophosphate, ferric pyrophosphate, iron lactate, ferrous gluconate, ferrous formate, ferric formate, potassium ferric ammonium oxalate, ferrous ascorbate, and sodium ferric edetate. The amount of the organic iron compound dissolved is 0.1 µM to 1 mM. When the amount dissolved is less than 0.1 µM, the effect of dissolving may not be sufficiently attained. Meanwhile, when the amount exceeds 1 mM, enhancement of the effect of dissolving may not be expected with increase only in the cost, and in addition, iron hydroxide and the like may be produced and precipitated. The amount of the organic iron compound dissolved is desirably 1 to 100 µM.

By dissolving an inorganic salt in the water with ozone-microbubbles generated, microbubbles can shrink and then stably exist as nanobubbles having a particle size of, for example, 10 to 500 nm, making it possible to contribute to maintenance of stability of permanganate ions in water. Specific examples of the inorganic acid include water soluble compounds, such as sodium chloride, potassium chloride, magnesium chloride, and magnesium sulfate. The amount of the inorganic salt dissolved is 1 to 300 mM. When the amount dissolved is less than 1 mM, the effect of dissolving may not be sufficiently attained. Meanwhile, when the amount exceeds 300 mM, enhancement of the effect of dissolving may not be expected with increase only in the cost. The amount of the inorganic salt dissolved is desirably 10 to 100 mM.

The dissolution of the prescribed amounts of the divalent manganese compound, the organic iron compound, and the inorganic salt in the water with ozone-microbubbles generated is desirably performed at an elapsed time of 10 minutes or longer after the redox potential of the water, which increases from an initial value of +300 mV to 500 mV by ozone-microbubbles being continuously generated, reaches at least +600 mV or higher, in that divalent manganese ions can be efficiently converted to permanganate ions (the increase in the redox potential comes to plateau at approximately +1000 mV). Also, after the prescribed amounts of the divalent manganese compound, the organic iron compound, and the inorganic salt are dissolved in the water with ozone-microbubbles generated, ozone is desirably continuously supplied in the form of microbubbles for at least 5 minutes, desirably at least 30 minutes, and more desirably at least 1 hour, in that permanganate ions can stably exist in the water over a long period of time.

The order of the dissolutions of the prescribed amounts of the divalent manganese compound, the organic iron compound, and the inorganic salt in the water with ozone-microbubbles generated is not particularly limited, and the compounds may be dissolved at once, or may be dissolved stepwise. The pH of the water with ozone-microbubbles generated in which the prescribed amounts of the divalent manganese compound, the organic iron compound, and the inorganic salt have been dissolved is desirably 3 to 10, and more desirably 5 to 9. The reason is as follows. Both the cases of too-high acidity and too-high alkalinity make the microbubbles and nanobubbles unstable and cause such bubbles to disappear while generating hydroxide radicals. In addition, the hydroxide radicals generated degrade the organic iron compound, and therefore permanganate ions may not exist in water stably over a long period of time. The adjustment of the pH may be appropriately achieved with hydrochloric acid or sodium hydroxide.

Although the water containing permanganate ions of the present invention and a water containing permanganate ions that is produced by dissolving a permanganate compound in water share a common point of containing permanganate ions, the two waters have significantly different properties from each other. Specifically, in the water containing permanganate ions of the present invention, permanganate ions exist in water stably over a long period of time at a concentration of 0.1 µM to 1 mM, typically at a concentration of 1 to 100 µM, and the half-life is 3 months or longer, for example, when the water filled in an airtight container under atmospheric pressure is stored under a temperature condition of 40°C. On the other hand, in the water containing permanganate ions that is produced by dissolving a permanganate compound in water, permanganate ions are reduced and disappear in a short period of time. In addition, as for the water containing permanganate ions of the present invention, for example, when the water filled in an airtight container under atmospheric pressure is cryopreserved for 6 months or longer under a temperature condition of -20°C and then naturally thawed at a normal temperature (25°C), the water recovers the state of the water containing permanganate ions before the cryopreservation. On the other hand, as for the water containing permanganate ions that is produced by dissolving a permanganate compound in water, since permanganate ions are reduced and disappear in a short period of time, when the water is cryopreserved and then thawed, the water does not recover the state of the water containing permanganate ions before the cryopreservation. Furthermore, when the electron spin resonance (ESR) spectrum of the water containing permanganate ions of the present invention is measured under a strongly acidic condition of, for example, pH 2, hydroxide radicals are detected (in addition, the magnitude of the peak is much larger than a magnitude of a peak of hydroxide radicals measured under the same condition as for an ozone water produced by generating ozone-microbubbles in water without dissolving a divalent manganese compound). On the other hand, the ESR spectrum of the water containing permanganate ions that is produced by dissolving a permanganate compound in water is measured under the same condition, singlet oxygen is detected. Although the reason why the differences occur is not exactly clear, the present inventor supposes the reason as follows. In the water containing permanganate ions of the present invention, owing to the oxidative effect of ozone supplied into water in the form of microbubbles, divalent manganese ions in the water do not stop in the trivalent or tetravalent form and oxidized to septivalent to produce permanganate ions, and the permanganate ions produced are attracted and trapped by an electrostatic effect around bubbles in the course of shrinkage of the microbubbles or around nanobubbles generated by the shrinkage, and held as one of components constituting an ion shell for a bubble. As a result, permanganate ions themselves are stabilized, and at the same time, the nanobubbles are also stabilized. The present inventor supposes that the existence of the organic iron compound around nanobubbles contributes to the fact that permanganate ions are firmly held as one of components constituting an ion shell for a bubble. Thus, the water containing permanganate ions of the present invention has not only an oxidative effect and the like of permanganate ions but also an oxidative effect and the like of ozone-nanobubbles, and can be utilized for various applications including one as an oxidant which is known as an application of each of them. For example, the water containing permanganate ions of the present invention is not irritating to mammal skin, has no oral acute toxicity to mammals, and has a sterilizing effect, a deodorant effect, and the like.

### Examples

Hereinunder, the present invention will be described in detail with reference to the examples. However, the present invention should not be construed as being limited to the following descriptions.

### Example 1:

Ozone-microbubbles were generated in distilled water using a commercially available microbubble generator that employs a two-phase flow swirling method (compact bubble generator manufactured by AQUAAIR Co. , Ltd.) that can generate microbubbles mainly having a particle size of 5 to 50 µm. An ozone gas that was prepared at a concentration of about 30 g/Nm³ using a commercially available oxygen source ozone generator was supplied at about 1 L/min to the microbubble generator so as to give an ozone concentration in water of about 10 mg/L. The redox potential of the water was confirmed to continuously increase from the initial value of about +500 mV by ozone-microbubbles being continuously generated, and then, at an elapsed time of 10 minutes after the redox potential reached +600 mV, 10 µM of iron ammonium citrate as an organic iron compound, 50 mM of sodium chloride as an inorganic salt, and 10 µM of manganese chloride as a divalent manganese compound were dissolved, and the pH was adjusted to 8 with sodium hydroxide. After that, when ozone-microbubbles were further continuously generated for 1 hour, the color of the water turned to light purple (pink), which suggested that permanganate ions be produced in the water. Since some sediment was recognized on the bottom of a beaker, the sediment was removed by filtration through a 450 nm membrane filter, and then the resulting filtrate was measured with a UV-visible-near infrared spectrophotometer (V-570 manufactured by JASCO Corporation: the same is applicable hereinbelow). As a result, the peak group of permanganate ion existed around 500-600 nm (when sodium chloride was dissolved in distilled water so as to give a salt concentration of 0.25% and then potassium permanganate was further dissolved therein to produce a water containing permanganate ions, and this water was measured under the same condition, it was confirmed that a peak group with the same shape existed at the same position: the same is applicable hereinbelow). The permanganate ion concentration of the thus produced water containing permanganate ions of the present invention was about 10 µM (the concentration was determined through conversion by comparing the height of the peak group of permanganate ion measured with the UV-visible-near infrared spectrophotometer with the height of the peak group of a water containing permanganate ions at a concentration of 20 µM that was produced as a standard solution with potassium permanganate: the same is applicable hereinbelow). It took about 30 minutes to produce 5L of the water containing permanganate ions of the present invention.

### Example 2:

Ozone-microbubbles were generated in distilled water using a commercially available microbubble generator that employs a pressurized dissolution method (A-02 manufactured by Shigenkaihatsukenkyujyo, Inc.) that can generate microbubbles mainly having a particle size of 5 to 50 µm. An ozone gas that was prepared at a concentration of about 30 g/Nm³ using a commercially available oxygen source ozone generator was supplied at about 1 L/min to the microbubble generator so as to give an ozone concentration in water of about 10 mg/L. The redox potential of the water was confirmed to continuously increase from the initial value of about +500 mV by ozone-microbubbles being continuously generated, and then, at an elapsed time of 10 minutes after the redox potential reached +600 mV, 10 µM of iron ammonium citrate as an organic iron compound, 50 mM of sodium chloride as an inorganic salt, and 10 µM of manganese nitrate as a divalent manganese compound were dissolved, and the pH was adjusted to 8 with sodium hydroxide. After that, when ozone-microbubbles were further continuously generated for 1 hour, the color of the water turned to light purple (pink), which suggested that permanganate ions be produced in the water. Since some sediment was recognized on the bottom of a beaker, the sediment was removed by filtration through a 450 nm membrane filter, and then the resulting filtrate was measured with a UV-visible-near infrared spectrophotometer. As a result, the peak group of permanganate ion existed around 500-600 nm. The permanganate ion concentration of the thus produced water containing permanganate ions of the present invention was about 8 µM. It took about 20 minutes to produce 10L of the water containing permanganate ions of the present invention.

### Example 3:

A water containing permanganate ions of the present invention was produced in the same manner as in Example 1, except that iron fulvate was dissolved in place of iron ammonium citrate.

### Example 4:

The half-life of permanganate ions of the water containing permanganate ions of the present invention produced in Example 1 was investigated in the case where the water filled in a PET bottle as an airtight container under atmospheric pressure was stored under a temperature condition of 40°C. As a result, the half-life measured was 3 months or longer (half or more of the permanganate ions remained after an elapsed time of 3 months).

### Example 5:

The water containing permanganate ions of the present invention produced in Example 1 filled in a PET bottle as an airtight container under atmospheric pressure was cryopreserved for 6 months or longer under a temperature condition of -20°C and then naturally thawed at a normal temperature (25°C). As a result, the water recovered the state of the water containing permanganate ions before the cryopreservation (even if the water was cryopreserved for 6 months before being thawed, the permanganate ion concentration was the same as that before the cryopreservation).

### Example 6:

The water containing permanganate ions of the present invention produced in Example 1 was allowed to stand in a dark place under a room temperature condition for 1 week. After that, DMPO (5,5-dimethyl-1-pyrroline N-oxide) which is a spin-trapping agent was added and hydrochloric acid was further added to measure the electron spin resonance (ESR) spectrum under a strongly acidic condition of pH 2. As a result, the spectrum of DMPO-OH which is a spin adduct (a spectrum that shows generation of hydroxide radicals) could be observed.

### Example 7:

Using the water containing permanganate ions of the present invention produced in Example 1 as a specimen, a primary skin irritation test was conducted using a rabbit conforming to the OECD Guidelines for the Testing of Chemicals 404. As a result, no irritation was observed.

### Example 8:

The water containing permanganate ions of the present invention produced in Example 1 was orally administered to a rat at a dose of 20 mg/kg for 14 days. As a result, no acute toxicity was observed.

### Example 9:

The sterilizing effect of the water containing permanganate ions of the present invention produced in Example 1 against a pathogenic bacterium, Salmonella enteritidis was investigated. As a result, an excellent sterilizing effect was observed.

### Example 10:

An appropriate amount of the water containing permanganate ions of the present invention produced in Example 1 was sprayed on cutting chips of PET bottles (industrial waste) which have an abnormal odor in summer. As a result, an excellent deodorant effect was shown.

### Example 11:

A water containing permanganate ions of the present invention was produced in the same manner as in Example 1, except that iron ammonium citrate, sodium chloride, and manganese chloride were dissolved at an elapsed time of 30 minutes after the redox potential of the water reached +600 mV by ozone-microbubbles being continuously generated in distilled water. As a result, the half-life of permanganate ions was prolonged as compared with the water containing permanganate ions of the present invention produced in Example 1.

### Comparative Example 1:

Potassium permanganate was dissolved in distilled water to produce a water containing about 10 µM of permanganate ions. The ESR spectrum of this water containing permanganate ions was measured under the same condition as in Example 6. As a result, in this water containing permanganate ions, the spectrum of a spin adduct, DMPO-OH could not be observed unlike in the water containing permanganate ions produced in Example 1, and alternatively the spectrum of singlet oxygen was observed. It was found from the above result that this water containing permanganate ions had significantly different properties from the water containing permanganate ions of the present invention produced in Example 1, although the two waters shared a common point of containing permanganate ions.

### Comparative Example 2:

The water containing permanganate ions produced in Comparative Example 1 was filled in a PET bottle as an airtight container under atmospheric pressure and stored under a temperature condition of 40°C. As a result, at an elapsed time of 1 month after the start of the test, the water color of light purple completely disappeared and the peak group of permanganate ion was not observed in a measurement with a UV-visible-near infrared spectrophotometer.

### Comparative Example 3:

The water containing permanganate ions produced in Comparative Example 1 filled in a PET bottle as an airtight container under atmospheric pressure was cryopreserved under a temperature condition of -20°C and then naturally thawed at a normal temperature (25°C). As a result, the water did not recover the state of the water containing permanganate ions before the cryopreservation.

### Comparative Example 4:

Using the water containing permanganate ions produced in Comparative Example 1 as a specimen, a primary skin irritation test was conducted using a rabbit conforming to the OECD Guidelines for the Testing of Chemicals 404. As a result, irritation was observed.

### Comparative Example 5:

The sterilizing effect of the water containing permanganate ions produced in Comparative Example 1 against a pathogenic bacterium, Salmonella enteritidis was investigated. As a result, the sterilizing effect was hardly observed.

### Comparative Example 6:

A water containing potassium permanganate was produced in the same manner as in Example 1, except that ozone was supplied by bubbling with a common aeration tube. This water containing permanganate ions was allowed to stand in a dark place under a room temperature condition. As a result, at an elapsed time of 1 day after the start of the test, the water color of light purple completely disappeared and the peak group of permanganate ion was not observed in a measurement with a UV-visible-near infrared spectrophotometer.

### Industrial Applicability

The present invention is industrially applicable in points of being capable of providing a water containing permanganate ions that exist stably over a long period of time and a method for producing the water.

## Claims

1. A water containing permanganate ions, which is produced by dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³.

2. The water containing permanganate ions according to claim 1, wherein the divalent manganese compound is at least one selected from the group consisting of manganese nitrate, manganese sulfate, and manganese chloride.

3. The water containing permanganate ions according to claim 1, wherein the organic iron compound is at least one selected from the group consisting of iron ammonium citrate, iron fulvate, iron acetate, heme iron, iron dextran, diethylenetriaminepentaacetic acid iron sodium salt, diethylenetriaminepentaacetic acid iron ammonium salt, ethylenediaminetetraacetic acid iron sodium salt, ethylenediaminetetraacetic acid iron ammonium salt, iron triethylenetetramine, dicarboxymethylglutamic acid iron sodium salt, ferrous citrate, iron sodium citrate, iron oxalate, ferrous succinate, iron sodium succinate citrate, ferrous pyrophosphate, ferric pyrophosphate, iron lactate, ferrous gluconate, ferrous formate, ferric formate, potassium ferric ammonium oxalate, ferrous ascorbate, and sodium ferric edetate.

4. The water containing permanganate ions according to claim 1, wherein the inorganic salt is at least one selected from the group consisting of sodium chloride, potassium chloride, magnesium chloride, and magnesium sulfate.

5. The water containing permanganate ions according to claim 1, wherein the generation of ozone-microbubbles is achieved by using a microbubble generator that can generate microbubbles having a particle size of 5 to 50 µm.

6. The water containing permanganate ions according to claim 5, wherein the microbubble generator is a microbubble generator that employs a two-phase flow swirling method or a pressurized dissolution method.

7. The water containing permanganate ions according to claim 1, wherein the dissolution of the divalent manganese compound, the organic iron compound, and the inorganic salt in the water with ozone-microbubbles generated is performed at an elapsed time of 10 minutes or longer after the redox potential of the water with ozone-microbubbles generated, which increases, reaches at least +600 mV.

8. The water containing permanganate ions according to claim 1, wherein when the water filled in an airtight container under atmospheric pressure is stored under a temperature condition of 40°C, the half-life of permanganate ions is 3 months or longer.

9. The water containing permanganate ions according to claim 1, wherein when the water containing permanganate ions filled in an airtight container under atmospheric pressure is cryopreserved for 6 months or longer under a temperature condition of -20°C and then naturally thawed at a normal temperature (25°C), the water recovers the state of the water containing permanganate ions before the cryopreservation.

10. The water containing permanganate ions according to claim 1, wherein the water containing permanganate ions is not irritating to mammal skin, has no oral acute toxicity to mammals, and has a sterilizing effect and a deodorant effect.

11. A method for producing a water containing permanganate ions, comprising dissolving 0.1 µM to 1 mM of a divalent manganese compound, 0.1 µM to 1 mM of an organic iron compound, and 1 to 300 mM of an inorganic salt in a water with ozone-microbubbles generated using an ozone gas at a concentration of 1 to 300 g/Nm³.

12. The method for producing a water containing permanganate ions according to claim 11, wherein the generation of ozone-microbubbles is achieved by using a microbubble generator that can generate microbubbles having a particle size of 5 to 50 µm.

13. The method for producing a water containing permanganate ions according to claim 11, wherein the dissolution of the divalent manganese compound, the organic iron compound, and the inorganic salt in the water with ozone-microbubbles generated is performed at an elapsed time of 10 minutes or longer after the redox potential of the water with ozone-microbubbles generated, which increases, reaches at least +600 mV.
